# EUROPÄISCHE PATENTANMELDUNG

(11) **EP 4 527 328 A1**
(43) Veröffentlichungstag der Anmeldung: **26.03.2025**
(21) Anmeldenummer: 23198816.3
(22) Anmeldetag: 21.09.2023
(51) Int. Cl.: A61B 18/14, A61B 18/00, A61B 18/02

(54) **ABLATIONSINSTRUMENT**

(71) Anmelder: Erbe Elektromedizin GmbH, 72072 Tübingen (DE)
(72) Erfinder: Adler, Marcus, 72393 Burladingen (DE); Karcher, Felix, 72072 Tuebingen (DE); Andel, Hanna, 72116 Moessingen (DE)
(74) Vertreter: Rüger Abel Patentanwälte PartGmbB

(57) **Zusammenfassung**

Mit dem erfindungsgemäßen Konzept ist ein Stecker (12) für ein Ablationsinstrument (11) geschaffen, der bis auf etwaige elektrische Leiter (25, 26) vollständig aus Kunststoff besteht und sich auf rationelle Weise mit wirtschaftlichen Fertigungsverfahren, beispielsweise in Spritzgusstechnik erzeugen lässt. Dazu trägt insbesondere die Stiftbaueinheit (30) bei, an der sowohl die Stifte (31, 32, 33), als auch der Sitz (43) für die Fluidleitung (20) und einer Anschlussfläche für die ebenfalls aus Kunststoff bestehende Gasweiche (45) ausgebildet sind. In dieser Bauform eignet sich das Ablationsinstrument (11) insbesondere als Einweginstrument, bei dessen Entsorgung keine wertvollen Materialien, sondern lediglich rationell herstellbare Kunststoffteile entsorgt oder recycelt werden müssen.

## Beschreibung

Die Erfindung betrifft ein Ablationsinstrument und eine Behandlungseinrichtung mit einem solchen. Die Erfindung betrifft dabei insbesondere CO₂-gekühlte Thermoablationsinstrumente, die biologisches, lebendes Gewebe durch Wärme oder Kälte devitalisieren. Zur Wärmeerzeugung im Gewebe können an dem Instrument Elektroden vorgesehen sein, die elektrischen Strom in das Gewebe leiten und dabei aber, um den Elektroden-Gewebe-Kontakt feucht und elektrisch leitfähig zu halten, von innen her gekühlt werden. Die Erfindung kann aber auch bei elektrodenlosen Instrumenten angewendet werden, die durch Kälte auf das Gewebe einwirken.

Aus der CN 1480103 A ist eine Kryosonde bekannt, die an ihrem distalen Ende eine mittels Joule-Thomson-Effekt gekühlte Instrumentenspitze aufweist. Das Instrument weist einen äußeren Schlauch und eine in dessen Lumen angeordnete Fluidleitung auf, die der Instrumentenspitze Fluid zuführt. Der Schlauch führt der Instrumentenspitze zugeflossenes Fluid in Gegenrichtung zurück. Der Schlauch ist an seinem von dem Patienten fern liegenden (proximalen) Ende in einem Handgriff gefasst. Dazu dient eine aus einem faserverstärkten Kunststoff bestehende Hülse, die sich durch eine niedrige Wärmeleitfähigkeit auszeichnet.

Auch die DE 10 2015 2005 367 A1 nutzt die Wärmeisolationseigenschaften eines faserverstärkten Kunststoffs an einer sogenannten Kryospitze.

Instrumente der eingangs genannten Art sind beispielsweise auch aus der EP 2630982 B1 bekannt. Dieses Instrument ist an seinem proximalen Ende mit einem Stecker versehen, der ein oder mehrere sich parallel von einem Steckergehäuse weg erstreckende Pins aufweist. Diese sind dazu vorgesehen, das Instrument mit kühlendem Fluid zu versehen. Von dem Stecker erstreckt sich ein Kabel mit einem elektrischen Stecker weg, der der elektrischen Versorgung des Instruments dient.

Es ist Aufgabe der Erfindung, ein mit CO₂ betreibbares Instrument zu schaffen, das eine einfache Herstellung gestattet und dabei dauerhaft zuverlässig ist.

Diese Aufgabe wird mit dem Ablationsinstrument sowie insbesondere auch mit der Behandlungseinrichtung nach Anspruch 15 gelöst:

Das erfindungsgemäße Ablationsinstrument kann, wie erwähnt, ein durch Wärme oder durch Kälte auf Gewebe wirkendes Instrument sein. In beiden Fällen weist es einen ein Lumen umschließenden Schlauch auf, der an seinem distalen Ende geschlossen ausgebildet und an seinem proximalen Ende mit einem Stecker verbunden ist. Dieser Stecker dient zumindest der Versorgung des Ablationsinstruments mit flüssigem, gasförmigem oder auch überkritischem Kohlendioxid (CO₂), wozu sich eine Fluidleitung von dem Stecker ausgehend durch das Lumen des Schlauchs erstreckt.

Der Stecker weist mindestens einen an die CO₂-Quelle anschließbaren Stift mit einem inneren Kanal auf, der fluidmäßig mit der Fluidleitung verbunden und an seinem äußeren von dem Stecker fern liegenden Ende geschlossen ist. Zwei an dem Stift im Abstand zueinander angeordnete Ringnuten nehmen Dichtungen, insbesondere ringförmige Dichtungen, beispielweise O-Ringe, auf. Die Dichtungen können als separate Bauelemente in die Ringnuten eingesetzt oder stoffschlüssig mit dem Stift verbunden sein. Dazu können sie in einem Co-Extrusionsverfahren zusammen mit dem Stift erzeugt worden sein. Zwischen den beiden O-Ringen ist eine in den Kanal führende Radialöffnung ausgebildet.

Erfindungsgemäß besteht der Stift aus einem faserverstärkten thermoplastischen Kunststoff, wobei als Kunststoff insbesondere Polycarbonat (PC) Polyamid (PA), Polybutylenterephthalat (PBT) oder auch Polyimiden (PI) in Frage kommen. Diese Kunststoffe sind vorzugsweise mittels in den Kunststoffkörper eingelagerter und darin vorzugsweise gleichmäßig verteilter sogenannter Kurzfasern verstärkt. Der Faseranteil kann zwischen 20 und 40 Gewichtsprozent liegen und beträgt vorzugsweise 30 Gewichtsprozent (Gew.%). Als Fasern kommen insbesondere Glasfasern infrage, wobei die Faserlänge vorzugsweise geringer ist als 3 mm, weiter vorzugsweise als 2 mm oder auch geringer als 1 mm. Andere vorzugsweise nichtmetallische Faserwerkstoffe, die gegenüber CO₂ inert sind und CO₂ auch nicht aufnehmen, können ebenfalls Anwendung finden. Es können Fasern aus organischem Material, bevorzugt aber Fasern aus anorganischem Material Anwendung finden. Die Dicke der Fasern liegt vorzugsweise bei etwa 5 µm bis 10 µm und beträgt vorzugsweise etwa 10pm.

Es hat sich gezeigt, dass derartige faserverstärkte Kunststoffe auch einer langfristigen Einwirkung (länger als 60 Minuten) von unter hohem Druck (mehr als 50 Bar) stehendem CO₂ gewachsen sind, während die gleichen Kunststoffe ohne Faserverstärkung nach einer gewissen Einwirkungszeit eine deutliche Dehnung zeigen. Eine solche Dehnung kann zu einer Längung des Stifts führen und zwar insbesondere im Bereich seiner Ringnut(en). Durch diese Längung kann es zu einem schlechten Sitz des Steckers an einer entsprechenden Fluidsteckdose kommen. Dies insbesondere, wenn in der Buchse ein an der Stirnfläche des Stifts anliegendes Element, z.B. ein Ventil oder Schalter oder schlicht eine ortsfeste Anlagefläche vorgesehen ist. Außerdem kann bei Längung des Stifts ein Stiftbruch oder Riss nicht ausgeschlossen werden. Die Erfindung hilft dem ab und gestattet einen Einsatz des Steckers und mit ihm des Instruments über mehrstündigen Betrieb hinweg. Beispielsweise kann das erfindungsgemäße Ablationsinstrument zur Tumorablation eingesetzt werden, bei dem es in einen Tumor eingestochen wird. An dem Instrument vorgesehene Elektroden dienen der Bestromung des Tumors und der Abtötung desselben durch Wärmeentwicklung. Um den dazu nötigen elektrischen Kontakt zwischen dem Tumorgewebe und den Elektroden aufrecht zu erhalten, werden die Elektroden von innen her gekühlt. Dazu dient das unter hohem Druck durch den Stecker geführte Kohlendioxid, dem der Stecker nunmehr trotz der Ausbildung seiner Stifte aus Kunststoff widersteht.

Zur Stromversorgung der optional an dem Ablationsinstrument vorhandenen Elektroden dienen elektrische Anschlussmittel, die an dem Stecker vorgesehen sein können. Ein solches Anschlussmittel kann beispielsweise durch einen oder mehrere Kontakte gebildet sein, die an einem oder mehreren Stiften des Steckers vorgesehen sein können. Bei einer bevorzugten Ausführungsform ist das Anschlussmittel durch eine elektrische Leitung mit einem elektrischen Stecker gebildet. Die Leitung kann ein Kabel sein, das sich von dem die Fluidverbindung zu dem Instrument herstellenden Stecker weg zu einem weiteren Stecker mit elektrischen Kontaktstiften erstreckt, die in einen dazu vorgesehenen elektrischen Generator einsteckbar sind.

Vorzugsweise weist der Stecker ein Steckergehäuse auf, in dem eine Stiftbaueinheit angeordnet ist. Die Stiftbaueinheit ist vorzugsweise als einstückiges Spritzgussteil ausgebildet, das einen in dem Steckergehäuse angeordneten Kopf und mindestens einen oder mehrere sich davon parallel zueinander weg erstreckende Stifte aufweist. Ist nur ein Stift vorhanden, kann dieser gestuft ausgebildet sein, um mehrere Fluidkanäle anzuschließen. Die Stiftbaueinheit ist zusammen mit den Stiften einstückig nahtlos aus dem gleichen faserverstärkten thermoplastischen Kunststoff ausgebildet. Sie vereint zusammen mit einer Gasweiche alle nötigen Funktionselemente, um den Schlauch und die Fluidleitung fluiddicht anzuschließen und eventuell vorhandene elektrische Leitungen aus dem Lumen des Schlauchs gasdicht heraus zu leiten. Außerdem leitet sie die koaxiale Gasführung des Instruments auf die parallele Gasführung der Stifte des Steckers um.

Die erfindungsgemäße Materialwahl gestattet es, insbesondere im Bereich der Nuten des jeweiligen Stifts, eine besonders geringe Wandstärke vorzusehen. Diese Wandstärke kann geringer sein als die in Stiftlängsrichtung gemessene Breite der betreffenden Nut. Es wird andererseits die Festlegung eines Kanaldurchmessers ermöglicht, der größer ist als die Wandstärke im Bereich der Nut. Dies vermeidet unerwünschte Drosselwirkungen im Gasweg bei der Gaszuleitung zu dem Instrument und somit eine gute Kühlwirkung an dem distalen Ende desselben.

Weitere Einzelheiten vorteilhafter Ausführungsformen der Erfindung sind Gegenstand der Figuren, der Zeichnung sowie der zugehörigen Beschreibung oder von Ansprüchen. In der Zeichnung zeigen:
Figur 1 eine Behandlungseinrichtung mit einem Ablationsinstrument, dessen Stecker an eine Steckdose eines Versorgungsgeräts angeschlossen ist, das mit einer CO₂-Gasquelle verbunden ist,
Figur 2 den Stecker des Ablationsinstruments nach Figur 1, in gesonderter Perspektivdarstellung und
Figur 3 einen Stift des Steckers nach Figur 1 und 2, in längsgeschnittener, ausschnittsweiser und vergrößerter Darstellung.

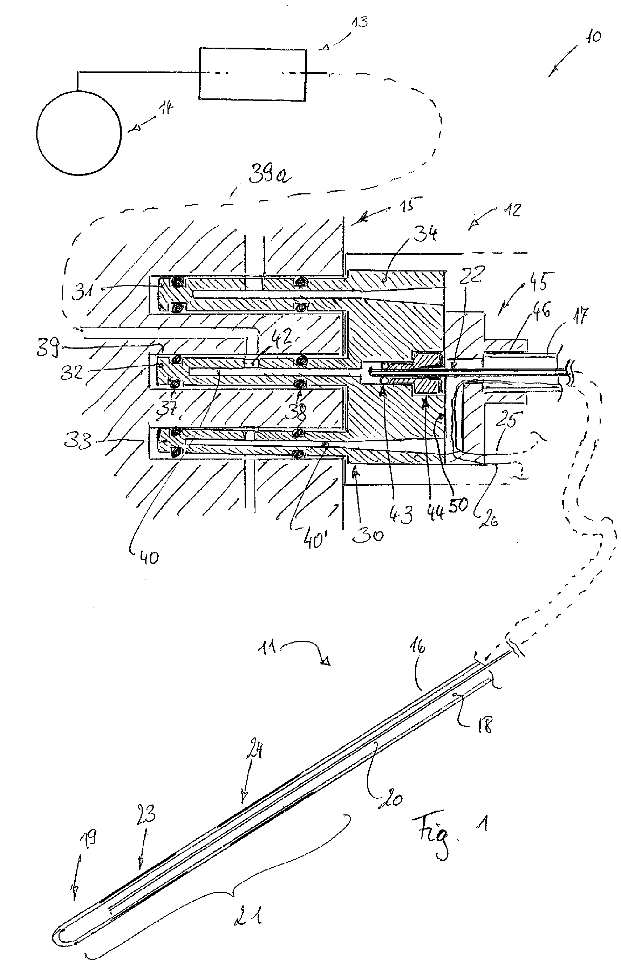

In Figur 1 ist eine Behandlungseinrichtung 10 veranschaulicht, die ein Ablationsinstrument 11 mit einem an seinem proximalen Ende vorgesehenen Stecker 12 umfasst. Außerdem gehört zu der Behandlungseinrichtung eine Gasversorgungseinheit 13, die aus einem Speicher 14 stammendes Kohlenstoffdioxidgas (CO₂) an eine Gassteckdose 15 leitet.

Das Ablationsinstrument 11 ist vorzugsweise als Einweginstrument ausgebildet, das nicht zum mehrmaligen Einsatz an verschiedenen Patienten und somit zur Wiederaufarbeitung vorgesehen ist. Prinzipiell ist es aber auch als Mehrweginstrument konzipierbar.

Das Ablationsinstrument 11 weist einen flexiblen, zum Beispiel durch den Arbeitskanal eines Endoskops in einem Patienten einführbaren Schlauch 16 auf, dessen proximales Ende 17 in dem Stecker 12 endet und gasdicht gefasst ist. Der Schlauch umschließt ein Lumen 18, das sich von dem proximalen Ende 17 bis zu dem distalen Ende 19 erstreckt, an dem der Schlauch 16 und somit das Lumen 18 geschlossen ist.

In dem Lumen 18 ist eine Fluidleitung 20 angeordnet, die der Zuleitung von CO₂ zu dem distalen Endbereich 21 des Instruments 11 und des Schlauchs 16 dient. Das proximale Ende 22 der Fluidleitung 20 ist wiederum in dem Stecker 12 fluiddicht gefasst. Die Fluidleitung 20 kann an ihrem Ende ein oder mehrere Auslassöffnungen aufweisen, um dort CO₂ austreten zu lassen, das dabei expandiert und dabei Wärme aufnimmt, d.h. kühlt.

In dem distalen Endbereich 21 können ein oder mehrere Elektroden 23, 24 angeordnet sein, die der Bestromung und dadurch Erwärmung von biologischem Gewebe dienen. Sie können über entsprechende elektrische Leitungen 25, 26 mit einem Stecker 27 verbunden sein, mit dem sie ein elektrisches Anschlussmittel 28 bilden. Der Stecker 27 kann ein, zwei oder mehrere elektrische Kontaktstifte 29 aufweisen, die dazu dienen, die Elektroden 23, 24 über die Leitungen 25, 26 mit Strom zu versorgen. Weiter können ein oder mehrere der Kontaktstifte 29 zur Instrumentenidentifikation eingerichtet sein und herangezogen werden.

Der Stecker 12 des erfindungsgemäßen Instruments 11 enthält eine Stiftbaueinheit 30 mit mindestens einem, vorzugweise aber mehreren Stiften 31, 32, 33, die sich parallel zueinander von einem Kopfstück 34 weg erstrecken, mit dem sie nahtlos einstückig ausgebildet sind.

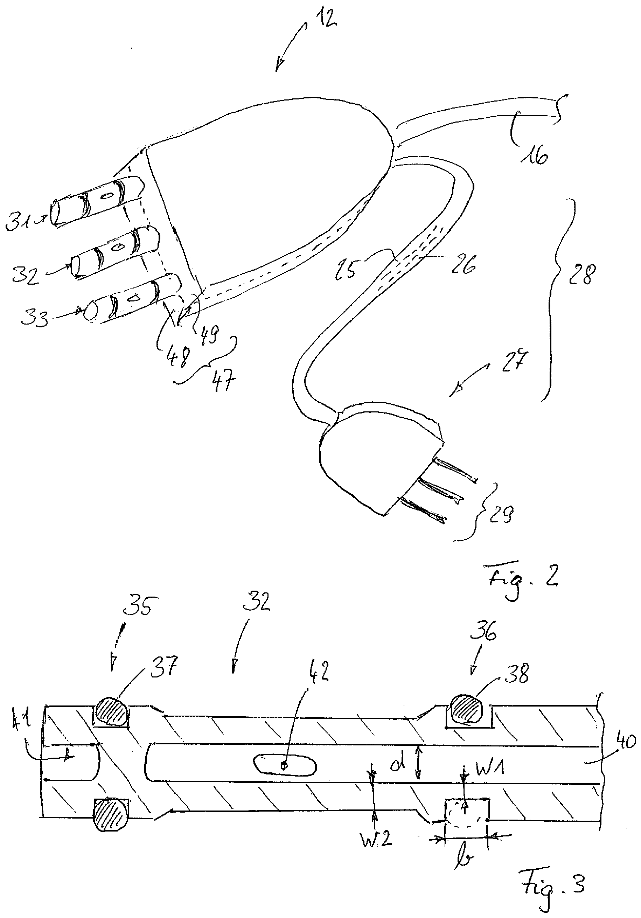

Die nachfolgende Beschreibung des Stifts 32 in Verbindung mit Figur 1 und 3 gilt entsprechend für die anderen Stifte 31, 33. Der Stift 32 weist einen länglichen, rohrartigen Grundkörper auf, an dessen Außenseite zwei Ringnuten 35, 36 in axialem Abstand zueinander angeordnet sind. Die Ringnuten 35, 36 dienen der Aufnahme von Dichtungselementen, beispielsweise O-Ringen 37, 38, mit denen der Stift 32 in einer entsprechenden Buchse 39 der Gassteckdose 15 abgedichtet positioniert ist. Der Stift 32 umschließt einen Kanal 40, der an dem von dem Stecker 12 fernliegenden Ende 41 geschlossen ist. Eine Radialöffnung 42 verbindet den Kanal 40 mit dem Innenraum der Buchse 39, wenn der Stecker 12 in die Gassteckdose 15 eingesteckt ist. Die Buchse 39 ist über einen Fluidkanal 39a mit der Gasversorgungseinheit 13 verbunden und wird über darüber mit unter Druck (z.B. zwischen 40 und 70 bar) stehendem CO₂ versorgt.

Bei der den Kanal 40 umschließenden Ringnut 36 weist der Stift 32 eine Wandstärke w1 auf, die geringer ist als die im Übrigen zwischen den Ringnuten 35, 36 zu messende Wandstärke w2. Außerdem ist die Wandstärke w1 vorzugsweise kleiner als die Nutbreite b, sowie ebenfalls kleiner als der Kanaldurchmesser d.

Der Kanal 40 mündet in einem Durchgang des Kopfstücks 34, der zugleich einen Sitz 43 für die abgedichtete Fassung des proximalen Endes 22 der Fluidleitung 20 bildet. Die Fluidleitung 20 ist dort mittels einer Dichtung gegen den Sitz 43 abgedichtet gehalten. An dem proximalen Ende 22 der Fluidleitung 20 ist ein Haltestück 44 mittels Klebstoffs gesichert.

An dem Kopfstück 34 ist außerdem eine Gasweiche 45 gehalten, die gasdicht auf der von den Stiften 31 bis 33 abliegenden, einen Aufnahmeplatz 50 bildenden z.B. ebenen Fläche des Kopfstücks 34 angeordnet ist. Dieser Aufnahmeplatz 50 bildet einen Sitz für die Gasweiche. Die Gasweiche dient der Ausleitung der Leitungen 25, 26 aus dem Lumen 18, der Umleitung des rückfließenden, entspannten CO₂ zu dem Stift und sichert zugleich das Haltestück 44 in dem Sitz 43. Das Haltestück 44 und die Gasweiche 45 können dabei einstückig ausgebildet sein. Der Schlauch 17 ist in einer Öffnung der Gasweiche 45 mittels Klebstoffs 46 gesichert. Die Gasweiche 45 enthält einen Kanal, über den das Lumen 18 mit einem Kanal 40' des Stifts 33 verbunden ist. Außerdem enthält die Gasweiche 45 eine Dichtung oder eine abdichtende Klebestelle für die Leitungen 25, 26, die der Kontaktierung der Elektroden 23, 24 dienen, um sie aus dem gasführenden Bereich des Steckers 12 herauszuführen.

Der Stecker 12 weist ein Steckergehäuse 47 auf, das zum Beispiel aus zwei Gehäuseschalen 48, 49 aus Kunststoff bestehen kann, wie es Figur 2 andeutet. Dieses Steckergehäuse 47 nimmt das Kopfstück 34 und die Gasweiche 45 auf. Die Stifte 31, 32, 33 ragen aus dem Gehäuse 47 heraus.

Die insoweit beschriebene Behandlungseinrichtung 10 arbeitet bei Anwendung des Ablationsinstruments 11 wie folgt:

Zur Devitalisierung von biologischem Gewebe wird das Ablationsinstrument 11 mit seinem distalen Endbereich 21 in das zu devitalisierende Gewebe, beispielsweise einen kompakten Lungentumor, eingestochen, sodass beide Elektroden 23, 24 in dem Tumor platziert sind. Bei Aktivierung des Instruments 11 leitet die Gasversorgungseinheit 13 dem Speicher 14 entnommenes Kohlenstoffdioxid unter einem kontrollierten Druck im Bereich von 40 Bar bis 70 Bar zu der Gassteckdose 15 und dort insbesondere zu der mittleren Buchse 39. Der entsprechende Gasleitungskanal 39a endet dabei in der Buchse zwischen den beiden O-Ringen 37, 38 des in die Buchse 39 eingesteckten Stifts 32.

Durch die Radialöffnung 42 und den Kanal 40 strömt nun Kohlendioxid zu dem Sitz 43 und von dort durch die Fluidleitung 20 bis in den distalen Endbereich 21 des Instruments, um diesen und mit ihm die Elektroden 23, 24 zu kühlen. Das CO₂ tritt dort aus der Fluidleitung 20 in das Lumen 18 über und expandiert dabei unter erheblicher Kälteentwicklung.

Gleichzeitig werden die Elektroden 23, 24 über den Stecker 27 oder ein sonstiges Anschlussmittel 28 mit elektrischem Strom versorgt, der den Tumor durchfließt und dabei erhitzt. Die Kühlung der Elektroden 23, 24 verhindert dabei die zu starke Erwärmung, sodass sich die maximale lokale Temperatur nicht an den Elektroden 23, 24, sondern in einiger Entfernung zu diesen einstellt. Dieser Behandlungsprozess kann eine erhebliche Zeitspanne von beispielsweise 15 Minuten in Anspruch nehmen. Auch kann der Behandler mehrere solcher Behandlungen miteinander kombinieren, indem er den betreffenden Tumor mehrmals behandelt oder weitere Tumore entsprechend behandelt (abladiert). Dies bedeutet, dass für einen erheblichen Gesamtzeitraum, der eine Stunde deutlich übersteigen kann, Kohlendioxid mit einem Druck von etwa 40 Bar bis 50 Bar oder mehr durch den Kanal 40 des Stifts 32 strömt. Dabei diffundiert Kohlendioxid in den Kunststoff hinein, aus dem der Stift 32 ausgebildet ist, was die Zugfestigkeit des Kunststoffs reduzieren kann. Durch die in dem Kunststoff vorhandenen Fasern, insbesondere Glasfasern, werden jedoch eine Aufweichung des Kunststoffs und insbesondere eine Dehnung im Bereich der Ringnut 36 wirksam unterbunden. Infolge dessen behält der Stift 32 seine Länge bei; weder dehnt er sich in Stiftlängsrichtung (Richtung des Kanals 40) aus, noch besteht Bruchgefahr im Bereich seiner geringen Wandstärken (beispielsweise im Bereich der Ringnut 36).

Mit dem erfindungsgemäßen Konzept ist ein Stecker 12 für ein Ablationsinstrument 11 geschaffen, der bis auf etwaige elektrische Leiter 25, 26 vollständig aus Kunststoff besteht und sich auf rationelle Weise mit wirtschaftlichen Fertigungsverfahren, beispielsweise in Spritzgusstechnik erzeugen lässt. Dazu trägt insbesondere die Stiftbaueinheit 30 bei, an der sowohl die Stifte 31, 32, 33, als auch der Sitz 43 für die Fluidleitung 20 und einer Anschlussfläche für die ebenfalls aus Kunststoff bestehende Gasweiche 45 ausgebildet sind. In dieser Bauform eignet sich das Ablationsinstrument 11 insbesondere als Einweginstrument, bei dessen Entsorgung keine wertvollen Materialien, sondern lediglich rationell herstellbare Kunststoffteile entsorgt oder recycelt werden müssen.

### Bezugszeichen:

- 10: Behandlungseinrichtung
- 11: Ablationsinstrument
- 12: Stecker
- 13: Gasversorgungseinheit
- 14: Speicher
- 15: Gassteckdose
- 16: Schlauch
- 17: proximales Ende des Schlauchs 16
- 18: Lumen
- 19: distales Ende des Schlauchs 16
- 20: Fluidleitung
- 21: distaler Endbereich des Schlauchs 16
- 22: proximales Ende der Fluidleitung 20
- 23, 24: Elektroden
- 25, 26: elektrische Leitungen
- 27: Stecker
- 28: elektrisches Anschlussmittel
- 29: elektrische Kontaktstifte
- 30: Stiftbaueinheit
- 31 ... 33: Stifte
- 34: Kopfstück
- 35, 36: Ringnuten
- 37, 38: O-Ringe
- 39: Buchse der Gassteckdose 15
- 39a: Fluidkanal
- 40: Kanal
- 41: Ende des Stifts 32
- 42: Radialöffnung
- w1: Wandstärke bei der Ringnut 36
- w2: Wandstärke zwischen den Ringnuten 35, 36
- b: Nutbreite
- d: Kanaldurchmesser des Kanals 40
- 43: Sitz
- 44: Haltestück
- 45: Gasweiche
- 46: Klebstoff
- 47: Gehäuse
- 48, 49: Gehäuseschalen
- 50: Aufnahmeplatz für die Gasweiche 45

## Patentansprüche

1. Ablationsinstrument (11):
mit einem ein Lumen (18) umschließenden Schlauch (16), der einen distalen Endbereich (21) und ein proximales Ende (17) aufweist und der an seinem distalen Ende (19) geschlossen ausgebildet ist und an seinem proximalen Ende (17) mit einem Stecker (12) verbunden ist,
mit einer sich durch das Lumen (18) von dem Stecker(12) zu dem distalen Endbereich (21) erstreckenden Fluidleitung (20),
wobei der Stecker (12) mindestens einen an eine CO₂-Gasversorgungseinheit (13) anschließbaren Stift (32) mit einem inneren Kanal (40) aufweist, der mit der Fluidleitung (20) verbunden ist,
wobei der Stift (32) zwei in einem Abstand zueinander angeordnete Ringnuten (35, 36) mit darin angeordneten Dichtungsringen (37, 38) aufweist, zwischen denen eine in den Kanal (40) führende Radialöffnung (42) ausgebildet ist,
**dadurch gekennzeichnet, dass** der Stift (32) aus einem faserverstärkten thermoplastischen Kunststoff besteht.

2. Ablationsinstrument nach Anspruch 1, **dadurch gekennzeichnet, dass** an dem Schlauch (16) an dessen distalen Endbereich (21) wenigstens eine Elektrode (23, 24) angeordnet ist, die mit einem an dem Stecker (12) vorgesehenen elektrischen Anschlussmittel (28) verbunden ist.

3. Ablationsinstrument nach Anspruch 1 oder 2, **dadurch gekennzeichnet, dass** der Stecker (12) ein Steckergehäuse (47) aufweist, in dem eine Stiftbaueinheit (30) angeordnet ist.

4. Ablationsinstrument nach einem der vorstehenden Ansprüche, **dadurch gekennzeichnet, dass** an der Stiftbaueinheit (30) mehrere parallel zueinander angeordnete Stifte (31, 32, 33) angeordnet sind, die nahtlos einstückig aus dem gleichen faserverstärkten thermoplastischen Kunststoff ausgebildet sind.

5. Ablationsinstrument nach einem der vorstehenden Ansprüche, **dadurch gekennzeichnet, dass** der in dem Stift (32) angeordnete Kanal (40) an seinem freien Ende (41) geschlossen ist.

6. Ablationsinstrument nach einem der vorstehenden Ansprüche, **dadurch gekennzeichnet, dass** der Stift (32) im Bereich der Nut (36) eine Wandstärke (w1) aufweist, die geringer ist als die Wandstärke (w2) außerhalb der Nut (36).

7. Ablationsinstrument nach einem der vorstehenden Ansprüche, **dadurch gekennzeichnet, dass** der Stift (32) im Bereich der Nut (36) eine Wandstärke (w1) aufweist, die geringer ist als die Hälfte der Wandstärke (w2) außerhalb der Nut (37).

8. Ablationsinstrument nach einem der vorstehenden Ansprüche, **dadurch gekennzeichnet, dass** der Stift (32) im Bereich der Nut (36) eine Wandstärke (w1) aufweist, die geringer ist als die in Stiftlängsrichtung gemessene Breite (b) der Nut (36).

9. Ablationsinstrument nach einem der vorstehenden Ansprüche 3 bis 8, **dadurch gekennzeichnet, dass** die Stiftbaueinheit (30) einen Sitz (43) zur abgedichteten Aufnahme der Fluidleitung (20) aufweist.

10. Ablationsinstrument nach einem der vorstehenden Ansprüche 3 bis 9, **dadurch gekennzeichnet, dass** die Stiftbaueinheit (30) einen Aufnahmeplatz (50) zur Aufnahme eines Gasweichenelements (45) aufweist, das einen Sitz zur abgedichteten Aufnahme des proximalen Endes (17) des Schlauchs (16) aufweist.

11. Ablationsinstrument nach einem der vorstehenden Ansprüche, **dadurch gekennzeichnet, dass** der Kunststoff PA, PC oder PBT ist.

12. Ablationsinstrument nach einem der vorstehenden Ansprüche, **dadurch gekennzeichnet, dass** der Kunststoff einen Faseranteil von 20 Gew.% bis 40 Gew.%, vorzugsweise 30 Gew.% aufweist.

13. Ablationsinstrument nach einem der vorstehenden Ansprüche, **dadurch gekennzeichnet, dass** die in dem Kunststoff vorhandenen Fasern Glasfasern sind.

14. Ablationsinstrument nach einem der vorstehenden Ansprüche, **dadurch gekennzeichnet, dass** die in dem Kunststoff vorhandenen Fasern eine Länge von unter 3mm, vorzugsweise unter 2mm, weiter vorzugsweise unter 1mm aufweisen.

15. Behandlungseinrichtung (10), die ein Ablationsinstrument (11) nach einem der vorstehenden Ansprüche, einen Gasspeicher (14) und eine CO₂-Gasversorgungseinheit (13) mit einer an den Stecker (12) des Ablationsinstruments (11) angepassten Steckdose (15) aufweist, die über einen Fluidkanal mit der CO₂-Gasversorgungseinheit (13) verbunden ist.
